# EUROPÄISCHE PATENTSCHRIFT

(11) **EP 1 437 128 B1**
(45) Veröffentlichungstag und Bekanntmachung des Hinweises auf die Patenterteilung: **09.07.2008**
(21) Anmeldenummer: 03000175.4
(22) Anmeldetag: 07.01.2003
(51) Int. Cl.: A61K 9/16, A61K 36/00

(54) **Zusammenstellung oder Kit von Pellets zur Zubereitung von chinesischen Arzneimitteltees und Verfahren zur Herstellung**
Composition or Kit from pellets for the preparation of medical Chinese tee and its method of preparation
Ensemble ou kit à base de pellets pour preparer du thé chinoise médicinal et sa méthode de préparation

(43) Veröffentlichungstag der Anmeldung: 14.07.2004
(73) Patentinhaber: Kaufmann, Michael, 50354 Hürth (DE); Neugebauer, Albert, 50259 Pulheim (DE); Neugebauer, Christoph, 50259 Pulheim (DE)
(72) Erfinder: Kaufmann, Michael, 50354 Hürth (DE); Neugebauer, Albert, 50259 Pulheim (DE); Neugebauer, Christoph, 50259 Pulheim (DE)
(74) Vertreter: Polypatent

(56) Entgegenhaltungen:
- US-A- 5 387 415
- US-A- 5 665 393
- PATENT ABSTRACTS OF JAPAN vol. 010, no. 350 (C-387), 26. November 1986 (1986-11-26) & JP 61 152238 A (AMANO JITSUGYO KK), 10. Juli 1986 (1986-07-10)
- FRANZBLAU S G ET AL: "Comparative in vitro antimicrobial activity of Chinese medicinal herbs." JOURNAL OF ETHNOPHARMACOLOGY. SWITZERLAND MAR 1986, Bd. 15, Nr. 3, März 1986 (1986-03), Seiten 279-288, XP009005314 ISSN: 0378-8741
- NETIEN G ET AL: "MEDICAMENTS HOMEOPATHIQUES" MEDICAMENTS HOMEOPATHIQUES. NOTIONS PRATIQUES DE PHARMACIE HOMEOPATIQUE, GALENICA, PARIS, TECHNIQUE ET DOCUMENTATION, FR, Bd. 16, 1986, Seiten 77-91, XP002131443

## Beschreibung

Die Erfindung betrifft ein Pellet enthaltend in einer Matrix einen Auszug eines Ausgangsstoffes von Tees, sowie Verfahren zur Herstellung der Pellets.

Die traditionelle chinesische Medizin ist eine Jahrtausende alte Erfahrungsmedizin, die auf überlieferten Erkenntnissen und Erfahrungen in der Heilung von Erkrankungen basiert. Die hierbei verwendeten Kräuter sind im chinesischen Arzneibuch dokumentiert. In der heute zunehmenden Phytotherapie wird festgestellt, dass die aus Kräutern gewonnenen Extrakte mit der Gesamtheit der darin enthaltenden Wirkstoffe vielfach eine bessere Beeinflussung von Erkrankungen bewirken als einzelne chemisch definierte Stoffe, die aus diesen Extrakten entwickelt wurden und in der Schulmedizin angewandt werden. Hierbei zeigte sich auch, dass bisher entwickelte und getestete Einzelstoffe oft mehr negative Nebenwirkungen zeigen als die Gruppe von Stoffen, die im Extrakt dieses Krauts vorliegen. Als Beispiel kann angeführt werden, dass Salixis cortex (die Weidenrinde) in der Kräuterkunde als Schmerzmittel besonders auch bei rheumatischen Schmerzen bekannt war. Die hieraus gewonnene Salicylsäure war über viele Jahre ein in bedeutendem Umfang eingesetztes Mittel zur Behandlung von Schmerzen. Es zeigte sich aber, dass Salicylsäure die Magenschleimhaut negativ beeinträchtigte. Heute kommen zunehmend Arzneimittel neu auf den Markt, die als Wirkstoff den Extrakt der Weidenrinde, und nicht mehr die reine Salicylsäure enthalten.

In der traditionellen chinesischen Medizin gibt es eine Vielzahl von Standardrezepturen an Heiltees. Zu mehr als 95 % werden jedoch individuelle Rezepturen verordnet.

PATENT ABSTRACTS OF JAPAN vol. 010, no. 350 (C-387), 26. November 1986 (1986-11-26) & JP 61 152238 A beschreibt ein Verfahren zu Herstellung eines Pellets enthaltend eine Matrix aus einer Mischung von Tee und Saccharide.

US-A-5 387 415 beschreibt ein Pellet enthaltend in einer Matrix chinesischen Arzneimittelsafts aus Aloe vera. Die Matrix besteht aus Kollagen oder Gelatine. Das Pellet kann als Instantform eines Ausgangsstoffes chinesischer Arzneimitteltees verwendet werden.

US-A-5 618 925 beschreibt ein Verfahren zu Herstellung eines Pellets aus Haifischknorpel zur Anwendung in der Traditionellen Chinesischen Medizin.

Im Stand der Technik sind die Vorschriften zur Zubereitung der einzelnen individuell verordneten chinesischen Tees (bzw. chinesischer Arzneimitteltees) sehr unterschiedlich. In der Regel verschreibt der Verordnende dem Patienten eine individuell abgestimmte Mischung pflanzlicher Drogen zur Anwendung in der traditionellen chinesischen Medizin, die in einer Apotheke gemischt wird. Der Patient muss diese Teemischung zuhause selbst zubereiten, indem er diesen Tee kocht. Bei einem üblichen Kochvorgang wird die Teemischung mit etwa 1 Liter Wasser übergossen und ca. eine halbe Stunde eingeweicht. Anschließend wird die Mischung zum Kochen gebracht und für ca. eine halbe Stunde leicht am Sieden gehalten. Danach wird die Mischung durch ein feines Sieb oder Baumwolltuch abgeseiht und die im Sieb oder Tuch zurückgehaltenen Kräuter werden nochmals mit ca. ½ Liter Wasser zum Kochen gebracht und etwa eine halbe Stunde unter leichtem Sieden gehalten. Danach wird erneut abgeseiht und die beiden Sude zusammen gegeben. Die sich ergebende Menge an Flüssigkeit wird eingeteilt und portionsweise über mehrere Tage, eventuell mit heißem Wasser verdünnt, verabreicht. Die Teeportionen für die weiteren Tage werden im Kühlschrank aufbewahrt, da der Tee sonst nicht haltbar ist. Bei der Zubereitung chinesischer Tees auf die herkömmliche Weise gilt es als wesentlich, dass die einzelnen pflanzlichen Ausgangstoffe zusammen gekocht werden, da eine als wesentlich erachtete gegenseitige Wechselwirkdung und Beeinflussung der Inhaltsstoffe angenommen wird.

Nachteilig an diesem Zubereitungsverfahren ist der hohe Zeitaufwand, relativ komplizierte Kochvorschriften, eine sehr starke Geruchsentwicklung, eine sehr begrenzte Haltbarkeit der gekochten chinesischen Tees, sowie die problematische Handhabung, da der fertige chinesische Tee mehrmals täglich in kleinen Portionen über den Tag verteilt getrunken werden soll. Da bisher nur jeweils die individuell zusammengestellte Teemischung als solche zubereitet wurde, ist der fertige chinesische Tee nur für den entsprechenden Patienten verwendbar.

Die Aufgabe der vorliegenden Erfindung ist es daher, die Zubereitung des chinesischen Arzneimitteltees für die Patienten zu vereinfachen, die sehr starke Geruchsentwicklung bei der Zubereitung durch den Patienten zu verringern, die Haltbarkeit der chinesischen Arzneimitteltees zu erhöhen und die Handhabung zu erleichtern. Weiterhin war es die Aufgabe, eine schnelle Trinkfertigkeit der chinesischen Tees bereitzustellen und den hohen Qualitätsstandard, der an die Ausgangsstoffe gestellt werden, aufrecht zu erhalten.

Die technische Aufgabe wird gelöst durch ein Pellet enthaltend in einer Matrix einen Auszug jeweils eines Ausgangsstoffes chinesischer Arzneimitteltees, wobei die Matrix einen Gerüstbildner aus hydrophilen Makromolekülen umfasst, und wobei der Ausgangsstoff chinesischer Arzneimitteltees ausgewählt ist aus der Gruppe pflanzlicher Ausgangsstoff, tierischer Ausgangsstoff, mineralischer Ausgangsstoff.

Die erfindungsgemäßen Pellets stellen eine Art Instantform jeweils eines Ausgangsstoffes dar, d.h. einer Droge, zur Anwendung in der traditionellen chinesischen Medizin. Dabei ist der Ausgangsstoff chinesischer Arzneimitteltees ausgewählt aus der Gruppe pflanzlicher Ausgangsstoff, tierischer Ausgangsstoff, mineralischer Ausgangsstoff zur Anwendung in der traditionellen chinesischen Medizin. So entspricht jeder einzelne pflanzliche Ausgangsstoff, d.h. jede einzelne pflanzliche Droge, Teilen einer bestimmten Pflanzenart oder -varietät oder der ganzen Pflanze. Die Pflanze ist dabei durch den wissenschaftlichen botanischen Namen (binominales System aus Gattung, Art, Varietät und Autor) definiert. In entsprechender Weise ist der tierische Ausgangsstoff definiert. Aus den einzelnen Pellets verschiedener Sorten, wobei jede Pelletsorte einem anderen Ausgangsstoff chinesischer Arzneimittteltees, insbesondere einem pflanzlichen Ausgangsstoff entspricht, lassen sich schnell individuelle Mischungen anfertigen. Je nach Rezept erhält der Patient eine Mischung von Pellets verschiedener Sorten oder auch eine Zusammenstellung von Pellets getrennt nach Sorten und kann seinen chinesischen Tee durch Aufbrühen der Pellets mit heißem Wasser schnell zubereiten.

Überraschenderweise wurde festgestellt, dass die chinesischen Tees, die aus Pellets der jeweiligen Ausgangsstoffe zubereitet wurden, d.h. bei denen die einzelnen Ausgangsstoffe getrennt abgekocht, in Pelletform überführt und erst nach dieser Behandlung gemischt und aufgebrüht wurden, die gleiche Qualität, die gleichen Eigenschaften und die gleiche Zusammensetzung aufweisen, wie die herkömmlich zubereiteten Tees, wobei die Ausgangstoffe zusammen gekocht werden. Dies wird in den Beispielen anhand pflanzlicher Ausgangsstoffe gezeigt.

Bei der Zubereitung chinesischer Tees aus den erfindungsgemäßen Pellets durch den Patienten entfällt der hohe Zeitaufwand, die starke Geruchsentwicklung und der Patient muss keine besonderen Kochvorschriften einhalten. Weiterhin weisen diese Pellets eine lange Haltbarkeit auf.

Dabei sind die Pellets enthaltend einen Auszug des gleichen Ausgangsstoffes chinesischer Tees, Pellets gleicher Sorte. Der Ausgangsstoff, aus dem der in den Pellets gleicher Sorte vorliegende Auszug gewonnen wird, ist höchstens einer von mehreren, d.h. mindestens zwei, vorzugsweise von mindestens vier, besonders bevorzugt von mindestens sechs, ganz besonders bevorzugt von mindestens zehn Ausgangsstoffen chinesischer Arzneimitteltees.

### Definitionen

"Chinesische Tee" werden aus pflanzlichen, tierischen und mineralischen Drogen (Ausgangsstoffen) hergestellt, wobei diese Drogen zur Anwendung in der traditionellen chinesischen Medizin bestimmt sind. Dabei sind die meisten Ausgangsstoffe pflanzlicher Herkunft und bestehen im Allgemeinen aus Pflanzen, Pflanzenteilen, Algen, Pilzen, wobei der pflanzliche Ausgangsstoff einer bestimmten Pflanzenart oder -varietät oder der ganzen Pflanze entspricht und die Pflanze durch den wissenschaftlichen botanischen Namen (binominales System aus Gattung, Art, Varietät und Autor) definiert ist. Die Begriffe chinesische Tees, chinesische Heiltees und chinesische Arzneimitteltees werden im Rahmen dieser Erfindung synonym verwendet. Weiterhin gibt es so genannte Präparata-Drogen, d.h. Ausgangsstoffe, die mit Honig gekocht wurden. Ein Beispiel hierfür ist der pflanzliche Ausgangsstoff Rehmannia radix, welcher gemäß der traditionellen chinesischen Medizin mit Honig gekocht werden kann, wobei Rehmannia radix präparata erhalten wird. Im Sinne der Erfindung kann ein Pellet daher entweder einen Auszug eines Ausgangsstoffes chinesischer Tees enthalten, oder einen Auszug eines Ausgangsstoffes chinesischer Tees enthalten, der mit Honig gekocht wurde. Somit werden auch Pellets bereit gestellt, die Präparata-Drogen enthalten.

Der "pflanzliche Ausgangsstoff" entspricht Teilen einer Pflanze oder der ganzen Pflanze. Dabei ist die Pflanze durch den wissenschaftlichen botanischen Namen, der dem binominalen System (Gattung, Art, Varietät und Autor) entspricht, eindeutig definiert. In diesem Sinne soll "pflanzlich" nicht nur die Gruppe der (photosynthetisch aktiven) Pflanzen, Algen und Flechten, sondern auch Pilze umfassen. Der "tierische Ausgangsstoff" entspricht Teilen eines Tieres oder des ganzen Tieres. Dabei ist das Tier ebenfalls durch den wissenschaftlichen zoologischen Namen, der dem binominalen System (Gattung, Art, Varietät und Autor) entspricht, eindeutig definiert.

Unter "Pellets" werden im wesentlichen symmetrisch und einheitlich ausgebildete Formkörper verstanden.

"Pellets einer Sorte" oder "Pellets gleicher Sorte" sind Pellets die einen Auszug aus dem gleichen Ausgangsstoff, insbesondere aus dem gleichen pflanzlichen Ausgangsstoff enthalten. Damit weisen die "Pellets einer Sorte" oder "Pellets gleicher Sorte" eine im wesentlichen gleiche Zusammensetzung auf, und unterscheiden sich von den Pellets einer anderen Sorte, d.h. Pellets, die aus einem anderen Ausgangsstoff hergestellt wurden, durch die Zusammensetzung der aus dem Ausgangsstoff stammenden Inhaltsstoffe. Mit dem Begriff "Sorte" soll nicht der Begriff "Pflanzensorte" aus der Pflanzenzucht verstanden werden, obwohl unter Umständen auch pflanzliche Ausgangsstoffe von Pflanzensorten als Ausgangsstoff chinesischer Arzneimitteltees verwendet werden könnten.

Ein "Dekokt" ist ein durch Abkochung von Ausgangsstoffen chinesischer Arzneimitteltees erhaltenes Produkt, wobei unter Einsatz einer bestimmten Menge Festsubstanz in Gewicht unter Zugabe von Wasser die gleiche Gewichtsmenge Dekokt erhalten wird. Unter dem Begriff "Auszug" soll im Sinne der Erfindung ein Dekokt eines Ausgangsstoffes verstanden werden. In diesem Sinne wird ein "Auszug eines Ausgangsstoffes" so definiert, dass dieser Auszug aus einem Ausgangsstoff, beispielsweise aus einem pflanzlichen Ausgangsstoff, d.h. einer bestimmten Pflanze oder einem bestimmten "Kraut" hergestellt wird. Gemäß der Erfindung soll der Ausgangsstoff, beispielsweise ein pflanzlicher Ausgangsstoff aus jenen ausgewählt werden, die zur Anwendung in der traditionellen chinesischen Medizin bestimmt sind, d.h. zur Zubereitung chinesischer Heiltees verwendet werden. In entsprechender Weise sind die Auszüge tierischer und mineralischer Ausgangsstoffe zu verstehen.

Der Begriff "Instant" besagt, dass ein Produkt in Pulver-, Granulat-, Pasten-, oder gemäß der Erfindung in Pelletform vorliegt, und beim Zusammenbringen mit einer Flüssigkeit rasch dispergiert und/oder gelöst wird, so dass schnell und einfach trinkfertige Zubereitungen erhalten werden. Der Begriff "Instant" bezeichnet nicht die trinkfertigen Zubereitungen, sondern nur die Vorstufe, z.B. in Form von Pellets.

In bevorzugter Weise stellen die Pellets die Instantform eines Ausgangsstoffes chinesischer Tees dar. Der Ausgangsstoff wurde vor Einbringung in das Pellet durch Abkochen vorbehandelt. Die Abkochung des Ausgangsstoffes stellt somit die Grundlösung dar, aus dem die Pellets gefertigt werden.

In einer weiteren bevorzugten Ausführungsform wird der Ausgangsstoff vor Einbringung in das Pellet durch Abkochen gewonnen, wobei die Ausgangsstoffe Drogen, d.h. pflanzliche, tierische oder mineralische Drogen zur Anwendung in der traditionellen chinesischen Medizin darstellen. In einer besonders bevorzugten Ausführungsform sind die pflanzlichen Ausgangsstoffe ausgewählt aus der Gruppe Pflanzen, Algen, Pilze, Flechte oder deren Teile. Besonders bevorzugt sind die pflanzlichen Ausgangsstoffe, ausgewählt aus den taxonomischen Gruppen Alismataceae, Companulaceae, Compositae, Cyperaceae, Dioscoreaceae, Labiatae, Leguminosae, Magnoliaceae, Polygonaceae, Ranunculaceae, Rutaceae, Rhamnaceae, Scrophulariaceae, Solanaceae, Umbelliferae.

In einer besonders bevorzugten Ausführungsform sind die pflanzlichen Ausgangsstoffe ausgewählt aus den in Tabelle 1 aufgeführten Ausgangsstoffen.

**Tabelle 1: Liste einer Reihe von pflanzlichen Ausgangstoffen, d.h. Pflanzen mit ihren entsprechenden chinesischen Namen (Pin Yin: 1. Spalte) und lateinischen Namen (2. Spalte), unter Erwähnung der Familien (3. Spalte).**

| **Pin Yin** | **Lateinische Bezeichnung** | **Familie** |
|---|---|---|
| Chuan Xin Lian | Andrographitis H. | Acanthaceae |
| Ze Xie | Alismatis Rh. | Alismataceae |
| Niu Xi (Huai) | Achyranthis Bidentatae R. | Amaranthaceae |
| Niu Xi (Chuan) | Cyanthulae R. | Amaranthaceae |
| Xian Mao | Curculiginis Rh. | Amaryllidaceae |
| Luo Bu Ma Ye | Apocyni Veneti Fol. | Apocynaceae |
| Shi Chang Pu | Acori Tatarinowii Rh. | Araceae |
| Tian Nan Xing | Arisaematis Rh. Prep. | Araceae |
| Ban Xia (Qing) | Pinelliae Rh. | Araceae |
| Ban Xia (Fa) | Pinelliae Rh. Prep. | Araceae |
| Ci Wu Jia | Acanthopanacis R. | Araliaceae |
| Ren Shen Ye | Ginseng Fol. | Araliaceae |
| San Qi (Tian Qi) | Notoginseng R. | Araliaceae |
| Xi Xing | Asari H. | Aristolochiaceae |
| Bai Wei | Cynanchi Atrati R. | Asclepiadaceae |
| Xu Chang Qing | Cynanchi Paniculati R. | Asclepiadaceae |
| Bai Qian | Cynanchi Stauntonii R. | Asclepiadaceae |
| Yin Yang Huo | Epimedii H. | Berberidaceae |
| Zi Cao (Ying) | Lithospermi R. | Boraginaceae |
| Sha Shen (Nan) | Adenophorae R. | Campanulaceae |
| Dang Shen | Codonopsis R. | Campanulaceae |
| Ban Bian Lian | Lobeliae Chinensis H. | Campanulaceae |
| Jie Geng | Platycodi R. | Campanulaceae |
| Ren Dong Teng | Lonicerae Caul. | Caprifoliaceae |
| Jin Yin Hua | Lonicerae FI. | Caprifoliaceae |
| Qu Mai | Dianthi H. | Caryophyllaceae |
| Tai Zi Shen | Pseudostellariae R. | Caryophyllaceae |
| Niu Bang Zi | Arctii Fr. | Compositae |
| Liu Ji Nu | Artemisaie Anomalae H. | Compositae |
| Qing Hao | Artemisiae Annuae H. | Compositae |
| Ai Ye | Artemisiae Argyi Fol. | Compositae |
| Ying Chen | Artemisiae Scopariae H. | Compositae |
| Zi Wan | Asteris R. | Compositae |
| Bai Zhu | Atractylodis Macrocephalae Rh. | Compositae |
| Cang Zhu | Atractylodis Rh. | Compositae |
| Mu Xiang | Auklandiae R. | Compositae |
| Hong Hua | Carthami FI. | Compositae |
| Ju Hua (Gong Ju) | Chrysanthemi FI. | Compositae |
| Ju Hua (Ye) | Chrysanthemi Indici FI. | Compositae |
| Xiao Ji | Cirsii H. | Compositae |
| Da Ji | Cirsii Japonici H. | Compositae |
| Han Lian Cao | Ecliptae H. | Compositae |
| Pei Lan | Eupatorii H. | Compositae |
| Kuan Dong Hua | Farfarae FI. | Compositae |
| Xuan Fu Hua | Inulae FI. | Compositae |
| Ze Lan | Lycopi H. | Compositae |
| Xi Xian Cao | Siegesbeckiae H. | Compositae |
| Pu Gong Ying | Taraxaci H. | Compositae |
| Cang Er Zi | Xanthii Fr. | Compositae |
| Tu Si Zi | Cuscutae S. | Convolvulaceae |
| Chui Pen Cao | Sedi H. | Crassulaceae |
| Da Qing Ye | Isatidis Fol. | Cruciferae |
| Ban Lan Gen | Isatidis R. | Cruciferae |
| Jie Zi (Bai) | Sinapis S. | Cruciferae |
| Mu Bie Zi | Momordicae S. | Curcubitaceae |
| Gua Lou | Trichosanthis Fr. | Curcubitaceae |
| Xiang Fu | Cyperi Rh. | Cyperaceae |
| Huang Yao Zi | Dioscoreae Bulbiferae L. Rh. | Dioscoreaceae |
| Bi Xie (Fen) | Dioscoreae Hypoglaucae Rh. | Dioscoreaceae |
| Shan Yao | Dioscoreae Rh. | Dioscoreaceae |
| Bi Xie (Mian) | Dioscoreae Septemlobae Rh. | Dioscoreaceae |
| Du Zhong | Eucommiae Co. | Eucommiaceae |
| Ba Dou | Crotonis Fr. | Euphorbiaceae |
| Jing Da Ji | Euphorbiae Pekinensis R. | Euphorbiaceae |
| Qin Jiao | Gentianae Macrophyllae R. | Gentianaceae |
| Long Dan | Gentianae R. | Gentianaceae |
| Zhu Ru | Bambusae in Taeniam Caul. | Gramineae |
| Yi Yi Ren | Coicis S. | Gramineae |
| Bai Mao Gen | Imperatae Rh. | Gramineae |
| Dan Zhu Ye | Lophatheri H. | Gramineae |
| Lu Gen | Phragmitis Rh. | Gramineae |
| She Gan | Belamcandae Rh. | Iridaceae |
| Hong Hua (Xi) | Croci Stigma | Iridaceae |
| Gui Pi (Rou Gui) | Cinnamomi Co. | Lauraceae |
| Gui Zhi | Cinnamomi Ram. | Lauraceae |
| Gui Xin | Cinnamomi Sin. Co. | Lauraceae |
| Wu Yao | Linderae R. | Lauraceae |
| He Huan Pi | Albiziae Co. | Leguminosae |
| Sha Wan Zi | Astragali Complanati S. | Leguminosae |
| Huang Qi | Astragali R. | Leguminosae |
| Jue Ming Zi | Cassiae Torae S. | Leguminosae |
| Zao Jiao Ci | Gleditsiae Spina | Leguminosae |
| Gan Cao | Glycyrrhizae R. | Leguminosae |
| Bai Bian Dou | Lablab Album S. | Leguminosae |
| Ge Gen | Puerariae R. | Leguminosae |
| Ku Shen | Sophorae Flavescentis R. | Leguminosae |
| Huai Jiao | Sophorae Fr. | Leguminosae |
| Huai Hua Mi | Sophorae jap. FI. | Leguminosae |
| Zhi Mu | Anemarrehnae Rh. | Liliaceae |
| Tian Dong | Asparagi R. | Liliaceae |
| Bei Mu (Chuan) | Fritillariae Cirrhosae Bulb. | Liliaceae |
| Bei Mu (Zhe) | Fritillariae Thumbergii Bulb. | Liliaceae |
| Bai He | Lilii Bulb. | Liliaceae |
| Mai Men Dong | Ophiopogonis R. | Liliaceae |
| Qi Ye Yi Zhi Hua | Paris Polyphyllae H. | Liliaceae |
| Yu Zhu | Polygonati Odorati Rh. | Liliaceae |
| Huang Jing | Polygonati Rh. | Liliaceae |
| Tu Fu Ling | Smilacis Glabrae Rh. | Liliaceae |
| Xing Yi Hua | Magnoliae FI. | Magnoliaceae |
| Hou Po | Magnoliae Officinalis Co. | Magnoliaceae |
| Wu Wei Zi | Schisandrae Fr. | Magnoliaceae |
| Chuan Lian Zi | Toosendan Fr. | Meliaceae |
| Bei Dou Gen | Menispermi Rh. | Menispermaceae |
| Fang Ji (Fen) | Stephaniae Tetrandrae R. | Menispermaceae |
| Huo Ma Ren | Cannabis S. | Moraceae |
| Sang Bai Pi | Mori Co. | Moraceae |
| Sang Ye | Mori Fol. | Moraceae |
| Sang Shen | Mori Fr. | Moraceae |
| Sang Zhi | Mori Ram. | Moraceae |
| Qian Shi | Euryales S. | Nymphaeaceae |
| He Ye | Nelumbinis Fol. | Nymphaeaceae |
| Lian Zi Xin | Nelumbinis Plumula | Nymphaeaceae |
| Lian Zi | Nelumbinis S. | Nymphaeaceae |
| Lian Qiao | Forsythiae Fr. | Oleaceae |
| Qin Pi | Fraxini Co. | Oleaceae |
| Nü Zhen Zi | Ligustri Lucidi Fr. | Oleaceae |
| Bai Ji | Bletillae Rh. | Orchidaceae |
| Shi Hu | Dendrobii H. Granulat | Orchidaceae |
| Tian Ma | Gastrodiae Rh. | Orchidaceae |
| Yan Hu So | Corydalis Rh. | Papaveraceae |
| Yuan Zhi | Polygalae R. | Polygalaceae |
| Bian Xu | Polygoni Avicularis H. | Polygonaceae |
| Hu Zhang | Polygoni Cuspidati Rh. | Polygonaceae |
| Shou Wu Teng | Polygoni Multiflori Caul. | Polygonaceae |
| He Shou Wu | Polygoni Multiflori R. | Polygonaceae |
| He Shou Wu (Zhi) | Polygoni Multiflori R. Prep. | Polygonaceae |
| Shen Da Huang | Rhei R. et Rh | Polygonaceae |
| Jin Qian Cao | Lysimachiae H. | Primaulaceae |
| Cao Wu (Sheng) | Aconiti Kusnezoffii R. | Ranunculaceae |
| Cao Wu (Zhi) | Aconiti Kusnezoffii R. Prep. | Ranunculaceae |
| Fu Zi | Aconiti Lateralis R. Prep. | Ranunculaceae |
| Chuan Wu (Sheng) | Aconiti R. | Ranunculaceae |
| Chuan Wu (Zhi) | Aconiti R. Prep. | Ranunculaceae |
| Sheng Ma | Cimicifugae Rh. | Ranunculaceae |
| Wei Ling Xian | Clematidis R. | Ranunculaceae |
| Mu Tong (Chuan) | Clematis Armandii Caul. | Ranunculaceae |
| Huang Lian | Coptidis Rh. | Ranunculaceae |
| Bai Shao | Paeoniae R. Alba | Ranunculaceae |
| Chi Shao | Paeoniae Rubra R. | Ranunculaceae |
| Bai Tou Weng | Pulsatillae chinensis R. | Ranunculaceae |
| Xian He Cao | Agrimoniae H. | Rosaceae |
| Xing Ren | Armeniacae amarum S. | Rosaceae |
| Mu Gua | Chaenomelis Fr. | Rosaceae |
| Shan Zha | Crataegi Fr. | Rosaceae |
| Pi Pa Ye | Eriobotryae Fol. | Rosaceae |
| Tao Ren | Persicae S. | Rosaceae |
| Wei Ling Cai | Potentillae Chinensis H. | Rosaceae |
| Yu Li Ren | Pruni S. | Rosaceae |
| Yue Ji Hua | Rosae Chinensis FI. | Rosaceae |
| Mei Gui Hua | Rosae Rugosae FI. | Rosaceae |
| Jin Ying Zi | Roseae Laevigatae Fr. | Rosaceae |
| Fu Pen Zi | Rubi Fr. | Rosaceae |
| Di Yu | Sanguisorbae (geröstet) R. | Rosaceae |
| Huo Xiang | Agastachis H. | Rubiaceae |
| Zhi Qiao | Aurantii Fr. | Rubiaceae |
| Zhi Shi | Aurantii Immaturus Fr. | Rubiaceae |
| Bai Xian Pi | Dictamni Co. | Rubiaceae |
| Wu Zhu Yu | Evodiae Fr. | Rubiaceae |
| Zhi Zi | Gardeniae Fr. | Rubiaceae |
| Lian Qian Cao | Glechomae H. | Rubiaceae |
| Chong Wei Zi | Leonuri Fuctus | Rubiaceae |
| Yi Mu Cao | Leonuri H. | Rubiaceae |
| Bo He | Menthae H. | Rubiaceae |
| Ba Ji Tian | Morindae Officinalis R. | Rubiaceae |
| Bai Hua She She Cao | Oldenlandiae H. | Rubiaceae |
| Zi Su Ye | Perillae Fol. | Rubiaceae |
| Zi Su Zi | Perillae Fr. | Rubiaceae |
| Huang Bai | Phellodendri Co. | Rubiaceae |
| Huo Xiang (Guang) | Pogostemonis H. | Rubiaceae |
| Xia Ku Cao | Prunellae Spica | Rubiaceae |
| Qian Cao | Rubiae R. | Rubiaceae |
| Dan Shen | Salviae Miltiorrhizae R. | Rubiaceae |
| Jing Jie | Schizonepetae H. | Rubiaceae |
| Ban Zhi Lian | Scutellariae H. | Rubiaceae |
| Huang Qin | Scutellariae R. | Rubiaceae |
| Gou Teng | Uncariae cum Uncis Ram. | Rubiaceae |
| Hua Jiao | Zanthoxyli Pericarpium | Rubiaceae |
| Chen Pi | Citri Reticulatae Pericarpium | Rutaceae |
| Long Yan Rou | Longan Arillus | Sapindaceae |
| Hu Huang Lian | Picrorhizae Rh. | Scrophulariaceae |
| Sheng Di Huang | Rehmanniae R. | Scrophulariaceae |
| Shou Di Huang | Rehmanniae R. Prep. | Scrophulariaceae |
| Xuan Shen | Scrophulariae R. | Scrophulariaceae |
| Di Gu Pi | Lycii Co. | Solanaceae |
| Gou Qi Zi | Lycii Fr. | Solanaceae |
| Bai Ying | Solani Lyrati H. | Solanaceae |
| Long Kui | Solani Nigri H. | Solanaceae |
| Bai Zhi | Angelicae Dahuricae R. | Umbelliferae |
| Du Huo | Angelicae Pubescentis R. | Umbelliferae |
| Dang Gui | Angelicae Sin. R. | Umbelliferae |
| Dang Gui Wei | Angelicae Sin. R. extr. | Umbelliferae |
| Chai Hu | Bupleuri R. | Umbelliferae |
| Chuan Xiong | Chuanxiong Rh. | Umbelliferae |
| She Chuang Zi | Cnidii Fr. | Umbelliferae |
| Xiao Hui Xiang | Foeniculi Fr. | Umbelliferae |
| Sha Shen (Bei) | Glehniae R. | Umbelliferae |
| Gao Ben | Ligustici sinensis Rh. | Umbelliferae |
| Qian Hu | Peucedani R. | Umbelliferae |
| Fang Feng | Saposhinikoviae R. | Umbelliferae |
| Bai Jiang Cao | Patriniae H. | Valerianaceae |
| Ma Bian Cao | Verbenae H. | Verbenaceae |
| Man Jin Zi | Viticis Fr. | Verbenaceae |
| Cao Dou Kou | Alpiniae Katsumandai S. | Zingiberaceae |
| Gao Liang Jiang | Alpiniae Officinarum Rh. | Zingiberaceae |
| Yi Zhi | Alpiniae Oxyphyllae Fr. | Zingiberaceae |
| Sha Ren | Amomi Fr. | Zingiberaceae |
| Dou Kou (Bai) | Amomi Rotundus Fr. | Zingiberaceae |
| Jiang Huang | Curcumae Longae Rh. | Zingiberaceae |
| Yu Jin | Curcumae R. | Zingiberaceae |
| E Zhu | Curcumae Rh. | Zingiberaceae |
| Cao Guo | Tsaoko Fr. | Zingiberaceae |
| Sheng Jiang | Zingerberis Recens Rh. | Zingiberaceae |
| Gan Jiang | Zingiberis Rh. | Zingiberaceae |
| Pao Jiang | Zingiberis Rh. Prep. | Zingiberaceae |

Besonders bevorzugt enthält der in Pellets gleicher Sorte enthaltene Auszug eines Ausgangsstoffes die Substanz oder die Kombination von Substanzen jeweils einer der in Tabelle 2 aufgeführten Gruppen. Die zur Zubereitung chinesischer Arzneimitteltees verwendeten Ausgangsstoffe werden erfindungsgemäß durch die in der Tabelle 2 genannten jeweils nach Ausgangsstoffen getrennten Gruppen von Hauptinhaltsstoffen charakterisiert. Dabei werden die pflanzlichen Ausgangsstoffe durch die Hauptinhaltsstoffe der Gruppen 1 bis 36 charakterisiert, die tierischen Ausgangsstoffe durch die der Gruppen 37 bis 42 und die mineralischen Ausgangsstoffe durch die der Gruppen 43 bis 46.

**Tabelle 2: Liste der Substanzgruppen, die in Pellets gleicher Sorte enthalten sind, d.h. in Pellets, die jeweils einem einzigen Ausgangsstoff entsprechen. Die Gruppen 1-36 entsprechen pflanzlichen Ausgangsstoffen; die Gruppen 37-42 entsprechen tierischen Ausgangsstoffen; und die Gruppen 43-46 entsprechen mineralischen Ausgangsstoffen.**

| **Gruppe** | **Inhaltsstoffe** |
|---|---|
| 1 | Alisol A, B, C (sind tetrazyklische Triterpene) |
| 2 | Byak-Angelicin, Byak-Angelicol, Imperatorin |
| 3 | n-Butyliden-Phthalid, D-2,4-Dihydrophthal-Säure, Sesquiterpene, Ferula-Säure, Nicotinsäure, Uracil, Sukrose |
| 4 | 1-2'-Methoxyphenyl-2,4-hexadien |
| 5 | Soyasaponin I, gamma-Aminobuttersäure, D-Glukose |
| 6 | Saikosaponine a-f, Stigmasterin, |
| 7 | 4-Hydroxy-3-Butylphthalide, Butylphthalide, Tetramethylpyrazin, Perlolyrine, Chuanxiongol, Ferulasäure |
| 8 | Taraxsteryl-Acetate, Friedelin, n-Butyl-Allophanate, Saponin, 7-Stigmasterin, 5,22-Stigmasterin, Inulin, Fruchtzucker, Aminosäuren, Alkaloide, Saponine |
| 9 | Ätherisches Öl, ß-Selinen, X-ß-Cyperone, Oleanolsäure, |
| 10 | Mannan, Phytinsäure, Allantoin, Dopamine |
| 11 | Glycyrrhizinat |
| 12 | Ziziphus saponin I, II, II, Jujubaside B (= Triterpensaponine), Fruchtzucker, Zucker, cAMP, CGMP, Aminosäuren |
| 13 | Betain, Zeaxanthin, Physalien, Vitamine B1 und C, Polysaccharide |
| 14 | Ätherisches Öl, ß-Eudesmol, Magnolol, Magnocurarine |
| 15 | Ätherisches Öl, 1-Menthol, I-Menthon |
| 16 | Paeonolide, Paeoniflorin, Benzoylpaeoniflorin, Benzoyl-Oxypaeoniflorin |
| 17 | Paeoniflorin |
| 18 | Berberin |
| 19 | Xanthone, Polygalitol |
| 20 | Lecithin |
| 21 | Pinicolsäure, 3ß-Hydroxylanosta-7,9-(11), 24-Trien-21 -Olsäure, Ergosterol, Polyporsäure |
| 22 | Catalpol, Dihydrocatalpol, Aucubin, Leonuride, Rehmannioside A, B, C, D, Melittoside, Rehmannin |
| 23 | Tanschinon I, Tanschinon IIA, Tanschinon IIB; Hydroxy-Tanschinon, Isotanschinon I und II |
| 24 | Baicalin, Baicalein, Woogonoside, Woogonin, Skulicapflavon I und II |
| 25 | Oleanolsäure |
| 26 | Aconitin |
| 27 | Methylchavicol |
| 28 | Amygdalin |
| 29 | Methylchavicol, Methyleugenol |
| 30 | Emodin |
| 31 | Zimtsäure, Zimtaldehyd |
| 32 | Coixol |
| 33 | Tetramethylpyrazin |
| 34 | Chlorogensäure |
| 35 | Lecithin |
| 36 | Aloe-Emodin, Emodin, Rhein |
| 37 | Buthotoxin, Trimethylamin, Betain, Taurin, Lecithin, Palmitinsäure |
| 38 | CaCO₃+E3583, CaPO₄, CaSO₄, Chitin, Si, Mg, AI |
| 39 | CaCO₃ Conchioline, Aminosäuren |
| 40 | CaCO₃ Leucin, Methionin, Alanin, Glycin, Glutaminsäure |
| 41 | Histaminartige Substanzen, Aminosäuren |
| 42 | Hypoxanthin, Lumbrofebrin, Lumbritin, Terrestro-lumbrylysin |
| 43 | CaSO₄ x 2H₂O |
| 44 | Fe₂O₃ |
| 45 | Fe₃O₄, Fe₂O₃, FeO |
| 46 | Mg₃ (Si₄O₁₀) (OH)₂ |

Besonders bevorzugt ist der Gerüstbildner ausgewählt aus der Gruppe Kollagen, Gelatine, fraktionierte Gelatine, Kollagenhydrolysate, Gelantinederivate, Pflanzenproteine, Pflanzenproteinhydrolysate, Elastinhydrolysate, Albumin, Agar-Agar, Gummi arabicum, Pektine, Traganth, Xanthan, natürliche und modifizierte Stärken, Dextrane, Dextrine, Maltodextrin, Chitosan, Alginate, Cellulosederivate, Zucker, Glycin, Lactose, Mannit, Polyvinylpyrrolidon, Polyacrylsäure, Polymere aus Methacrylsäure, Polymere aus Methacrylsäureestern, Celluloseactatphthalat, Hydroxypropylmethylcellulosephthalat, azovernetzte Polymethacrylate, Polyurethan-Zucker-Copolymere, Galactomannanderivate, Polysaccharide, Mono-, Di-, und Triglyceride und deren Mischungen.

Es ist bevorzugt, dass der Gerüstbildner ausgewählt wird aus der Gruppe Oligosaccharide, Polysaccharide, Dextran, Dextrin, Maltodextrin. In einer besonders bevorzugten Ausführungsform wird Maltodextrin oder Dextran oder deren Mischung als Gerüstbildner im Pellet gewählt. Ganz besonders bevorzugt wird Dextran als Gerüstbildner im Pellet verwendet.

Weiterhin wird bevorzugt, dass die Größe der enthaltenden gefriergetrockneten Pellets 1 bis 10 mm, vorzugsweise 2 bis 5 mm beträgt.

Die Erfindung betrifft weiterhin eine Instantzubereitung chinesischer Arzneimitteltees, enthaltend gefriergetrocknete Pellets, sowie die Verwendung der erfindungsgemäßen Pellets oder der Instantzubereitung zur Zubereitung von chinesischen Arzneimitteltees. Weiterhin betrifft die Erfindung ein Verfahren zur Zubereitung von chinesischen Tees, wobei die erfindungsgemäßen Pellets oder die erfindungsgemäße Instantzubereitung in Wasser gelöst werden.

Insbesondere betrifft die Erfindung eine Zusammenstellung oder ein Kit von Pellets zur Zubereitung von chinesischen Arzneimitteltees, wobei Pellets der gleichen Sorte in einer Matrix einen Auszug jeweils eines Ausgangsstoffes enthalten, wobei der Ausgangsstoff ausgewählt ist aus einer Gruppe von verschiedenen Ausgangstoffen chinesischer Arzneimitteltees, wobei die Zusammenstellung mindestens zwei verschiedene jeweils voneinander getrennt vorliegende Pelletsorten enthält. Die in der erfindungsgemäßen Zusammenstellung enthaltenden Pellets sind gemäß der vorliegenden Erfindung, wie oben ausgeführt, charakterisiert. In einer bevorzugten Ausführungsform enthält die Zusammenstellung mindestens vier, besonders bevorzugt mindestens sechs, ganz besonders bevorzugt mindestens zehn verschiedene jeweils voneinander getrennt vorliegende Pelletsorten. Mit der erfindungsgemäßen Zusammenstellung lassen sich je nach Rezeptur des Verordnenden unterschiedliche individuell auf den Patienten abgestimmte Mischungen chinesischer Heiltees besonders einfach herstellen.

Diese Zusammenstellung von Pellets verschiedener Pelletsorten ermöglicht es dem Apotheker eine spezielle Mischung von Pellets verschiedener Sorten zusammenzustellen, die von dem Patienten durch Aufbrühen mit Wasser sehr schnell in einen chinesischen Tee zubereitet werden kann, dessen Rezeptur speziell auf die spezifischen Bedürfnisse des Patienten abgestimmt wurde.

In einer bevorzugten Ausführungsform sind in der Zusammenstellung die Pellets einer Sorte jeweils luftdicht verpackt. Die Pellets sind auf diese Weise sehr lange haltbar. Auch nach Öffnen der Verpackung sind die erfindungsgemäßen Pellets einige Tage an der Luft haltbar.

In einer weiteren bevorzugten Ausführungsform enthält die Zusammenstellung ein Dosierungsset, d.h. ein Kunststoffteil mit mehreren Vertiefungen, in die dann der Apotheker die auf den Patienten abgestimmte Mischung von Pellets verschiedener Sorten einfüllt. Nach dem Einfüllen der jeweiligen Mischung in die Vertiefungen, wird das Dosierungsset beispielsweise mit Folie abgedeckt bzw. abgeklebt. Die Pelletmischung, die in jeweils einer Vertiefung dieses Dosierungssets an den Patienten abgegeben wird, stellt die Mischung für einen speziellen chinesischen Arzneimitteltee für einen Tag, d.h. eine Tagesdosis, dar.

Weiterhin betrifft die Erfindung eine Mischung der erfindungsgemäßen Pellets, wobei die Mischung mindestens zwei verschiedene Pelletsorten enthält und wobei eine Pelletsorte so definiert ist, dass der in einem Pellet vorliegende Auszug aus jeweils einem Ausgangsstoff chinesischer Tees gewonnen wurde.

Es wird zudem ein Verfahren beansprucht zur Zubereitung von chinesischen Arzneimitteltees, wobei die Ausgangsstoffe dieser chinesischen Arzneimitteltees jeweils getrennt abgekocht werden, das jeweilige erhaltende Dekokt mit Gerüstbildner versetzt wird, pelletiert wird und anschließend gefriergetrocknet wird, um nach Ausgangsstoffen getrennte Pelletsorten zu erhalten, und anschließend eine Mischung von Pellets verschiedener Sorten in Wasser gelöst wird, um chinesischen Arzneimitteltee herzustellen.

Die technische Aufgabe wird weiterhin gelöst durch ein Verfahren zur Herstellung von chinesischen Arzneimitteltees in Instantform auf Basis von Pellets enthaltend Auszüge von Ausgangsstoffen chinesischer Arzneimitteltees, wobei der Ausgangsstoff chinesischer Arzneimitteltees ausgewählt ist aus der Gruppe pflanzlicher Ausgangsstoff, tierischer Ausgangsstoff, mineralischer Ausgangsstoff, umfassend die Schritte:
a) Zubereitung eines Dekoktes aus den Ausgangsstoffen chinesischer Arzneimitteltees,
b) Lösen und/oder Dispergieren eines Gerüstbildners im Dekokt und Erhalten einer Dekokt/Gerüstbildner-Mischung,
c) Gefrieren der in Schritt b) erhaltenen Dekokt/Gerüstbildner-Mischung durch tropfenweises Einbringen in eine tiefkalte kryogene Flüssigkeit und Erhalten von Pellets,
d) Gefriertrocknung der erhaltenen Pellets.

Nach dem Abkochen lässt man das Dekokt abkühlen. In dem erfindungsgemäßen Verfahren wird jeweils ein Dekokt eines Ausgangsstoffes getrennt von anderen zubereitet, um die erfindungsgemäßen Pellets herzustellen. In einem bevorzugten Verfahren werden nach Schritt a) Feststoffe entfernt, vorzugsweise durch Filtration. Die Filtration kann unter Verwendung eines feinen Siebes oder eines Baumwolltuches oder Filters erfolgen. Vor oder nach der Filtration kann das Dekokt verdünnt werden.

In einem weiteren bevorzugten Verfahren handelt es sich bei den zu verarbeitenden Ausgangsstoffen um pflanzliche, tierische oder mineralische Drogen zur Anwendung in der traditionellen chinesischen Medizin. Besonders bevorzugt werden als pflanzliche Ausgangsstoffe Pflanzen, Algen, Pilze, Flechten oder deren Teile verwendet. Besonders bevorzugt werden die pflanzlichen Ausgangsstoffe ausgewählt aus den taxonomischen Gruppen Alismataceae, Companulaceae, Compositae, Cyperaceae, Dioscoreaceae, Labiatae, Leguminosae, Magnoliaceae, Polygonaceae, Ranunculaceae, Rutaceae, Rhamnaceae, Scrophulariaceae, Solanaceae, Umbelliferae.

In bevorzugter Weise werden die Ausgangsstoffe ausgewählt aus den in der Tabelle 1 aufgeführten Ausgangsstoffen.

Vorzugsweise sind die Ausgangsstoffe definiert durch die Substanzen oder die Kombination von Substanzen jeweils einer der in Tabelle 2 aufgeführten Gruppen.

In einem weiteren bevorzugten Verfahren beträgt das Gewichtsverhältnis der eingesetzten Festsubstanz der Ausgangsstoffe und Wasser zu dem in Schritt a) erhaltenen Dekokte 0,8:1 bis 1,2:1, bevorzugt 0,9:1 bis 1,1:1 und besonders bevorzugt um 1:1.

Weiterhin ist bevorzugt, dass der Feststoffgehalt des in Schritt a) erhaltenen und in Schritt b) weiter zu verarbeitenden Dekoktes 1 bis 20 Gew.-%, vorzugsweise 1 bis 10 Gew.-% beträgt.

In einem weiteren, besonders bevorzugten Verfahren wird der Gerüstbildner ausgewählt aus der Gruppe Kollagen, Gelatine, fraktionierte Gelatine, Kollagenhydrolysate, Gelantinederivate, Pflanzenproteine, Pflanzenproteinhydrolysate, Elastinhydrolysate, Albumin, Agar-Agar, Gummi arabicum, Pektine, Traganth, Xanthan, natürliche und modifizierte Stärken, Dextrane, Dextrine, Maltodextrin, Chitosan, Alginate, Cellulosederivate, Zucker, Glycin, Lactose, Mannit, Polyvinylpyrrolidon, Polyacrylsäure, Polymere aus Methacrylsäure, Polymere aus Methacrylsäureestern, Celluloseactatphthalat, Hydroxypropylmethylcellulosephthalat, azovernetzte Polymethacrylate, Polyurethan-Zucker-Copolymere, Galactomannanderivate, Polysaccharide, Mono-, Di-, und Triglyceride und deren Mischungen.

In einem ganz besonders bevorzugten Verfahren wird ein Gerüstbildner verwendet, ausgewählt aus der Gruppe Oligosaccharide, Polysaccharide, Dextran, Dextrin, Maltodextrin, sowie Mischungen derselben. In einer besonders bevorzugten Ausführungsform wird Maltodextrin oder Dextran, oder deren Mischung als Gerüstbildner verwendet. Besonders bevorzugt wird Dextran als Gerüstbildner verwendet.

Weiterhin ist ein Verfahren bevorzugt, wobei nach Lösen des Gerüstbildners in Schritt b) 2 bis 50 Gew.-%, vorzugsweise 5 bis 40 Gew.-% Gerüstbildner in der Dekokt-Gerüstbildnermischung vorliegt. Vorzugsweise wird als kryogene Flüssigkeit flüssiger Stickstoff zum Gefrieren der in Schritt b) erhaltenen Mischung verwendet.

In einem weiteren bevorzugten Verfahren beträgt die Größe der erhaltenen gefriergetrockneten Pellets 1 bis 10 mm, vorzugsweise 2 bis 5 mm.

Für die Gefriertrocknung der erhaltenen Pellets wird bevorzugt die Rotationsgefriertrocknung angewendet werden. Die einheitliche und kugelförmige Pelletform der erfindungsgemäßen Pellets führt vorteilhafterweise zu extrem kurzen Trocknungszeiten.

Weiterhin betrifft die Erfindung eine Instantform eines Ausgangsstoffes chinesischer Arzneimitteltees in Form von gefriergetrockneten Pellets erhältlich nach dem oben beschriebenen erfindungsgemäßen Verfahren.

Ein besonderer Vorteil des erfindungsgemäßen Verfahrens besteht darin, dass nach Schritt a) ein Dekokt aus pflanzlichen, tierischen oder mineralischen Ausgangsstoffen als stark konzentrierte Lösung erhalten wird und somit ein relativ geringes Volumen aufweist. Zu dieser stark konzentrierten Lösung wird der Gerüstbildner gegeben und aufgelöst oder dispergiert. Durch diese Vorgehensweise wird eine gleichmäßige Form der Tropfen in Schritt c) sowie eine relativ einheitliche Größe der tiefgefrorenen Pellets erhalten. Weiterhin ergeben sich durch diese Verfahrensschritte kurze Trocknungszeiten, die durch die Anwendung eines Rotationsgefriertrockners weiter verkürzt werden können.

Mit dem erfindungsgemäßen Verfahren können aus den Dekokten der einzelnen Ausgangsstoffe gefriergetrocknete Pellets erzeugt werden. Insbesondere beinhaltet die Herstellung der Pellets verschiedener Sorten, d.h. Pellets, die von unterschiedlichen Ausgangsmaterialien stammen, einen Schritt des Abkochens. Im bisherigen Stand der Technik wurden alle Ausgangsstoffe für einen speziell zusammengestellten chinesischen Tee zusammen abgekocht. Im Stand der Technik wird davon ausgegangen, dass das Abkochen der bereits gemischten Ausgangsstoffe aufgrund der Wechselwirkung der Inhaltsstoffe von großer Bedeutung ist. Im Gegensatz dazu werden gemäß der vorliegenden Erfindung die einzelnen Ausgangsstoffe chinesischer Tees getrennt abgekocht und anschließend jeweils getrennt nach Ausgangsstoffen in eine getrocknete Pelletform überführt. Die bereits vorbehandelten getrockneten Pellets können dann in der Rezeptur individuell gemischt werden, um aus dieser Mischung getrockneter Pellets, den speziellen chinesischen Tee aufzubrühen.

In den folgenden Beispielen wird gezeigt, dass ein chinesischer Tee, der hergestellt wird durch Abkochen einer Mischung der verschiedenen pflanzlichen Bestandteile eines chinesischen Tees, sich hinsichtlich der Zusammensetzung und Menge einzelner Substanzen nicht von einer Mischung unterscheidet, bei der die einzelnen pflanzlichen Bestandteile zunächst getrennt gekocht wurden und erst nach dem Abkochen gemischt wurden.

Weiterhin wurde in den folgenden Beispielen gezeigt, dass die Inhaltsstoffe eines Dekoktes aus einem bestimmten pflanzlichen Ausgangsstoff sich hinsichtlich der Zusammensetzung und Menge der Inhaltsstoffe nicht von einer Lösung unterscheidet, die durch Aufbrühen von Pellets in heißem Wasser, hergestellt wurde, wobei die Pellets nach dem erfindungsgemäßen Verfahren aus einem Dekokt des gleichen pflanzlichen Bestandteiles zubereitet wurde.

Die beiden Vergleiche zeigen, dass gemäß der vorliegenden Erfindung chinesische Tees zubereitet werden können, wobei entgegen der im Stand der Technik üblichen Verfahrensweise, die einzelnen pflanzlichen Ausgangsstoffe zunächst getrennt gekocht werden, in Pelletform überführt und gefriergetrocknet werden, bevor aus einer Mischung von Pellets verschiedener Sorten ein chinesischer Arzneimitteltee zubereitet wird. Dies gilt in entsprechender Weise auch für die tierischen oder mineralischen Ausgangsstoffe chinesischer Heiltees.

Die Ergebnisse zeigen klar, dass die erfindungsgemäßen Pellets die hohen Ansprüche an die Qualität der pflanzlichen Ausgangsstoffe und an die Zubereitung der chinesischen Tees auch durch die erfindungsgemäßen Abwandlungen, wie getrenntes Abkochen und Überführen in eine getrocknete Pelletform, aufrecht erhalten werden können.

### Beispiele

In den nachfolgenden Beispielen werden die einzelnen pflanzlichen Ausgangsstoffe entweder als Mischung oder einzeln, und entweder entsprechend dem erfindungsgemäßen Verfahren oder in herkömmlicher Weise behandelt.

### Beispiel 1: Zubereitung eines Dekoktes, Analyse mit HPLC

Die pflanzlichen Ausgangsstoffe werden in einen Topf gegeben und mit Wasser bedeckt und anschließend 30 Minuten einweichen lassen. Danach wird kurz aufgekocht und 30 Minuten unter Sieden gehalten. Der Sud wird anschließend durch ein Sieb gegeben. Der Rückstand aus dem Sieb wird mit einem halben Liter Wasser erneut aufgekocht und nochmals 30 Minuten unter Sieden gehalten und anschließend durch ein Sieb gegeben. Anschließend werden die beiden Sude zu einem Dekokt vereint. Zum Vergleich der Dekokte nach den verschiedenen Verfahren werden die Testlösungen auf ein definiertes einheitliches Volumen gebracht und anschließend noch gefiltert, um störende Schwebstoffe zu entfernen.

Die in den Beispielen 2 bis 5 beschriebenen Produkte, Dekokt bzw. aufgelöstes Pellet, wurden mittels HPLC analysiert. In der HPLC wurden folgenden Lösungsmittel verwendet: Lösungsmittel A: 190 ml Acetonitril, 10 ml H₃PO₄, 800 ml H₂O; Lösungsmittel B: 1180 ml Acetonitril, 20 ml H₃PO₄, 800 ml Methanol. Die Lösungsmittel A und B wurden gegeneinander ansteigend bei der HPLC benutzt.

### Beispiel 2: Gemeinsame Zubereitung von pflanzlichen Ausgangsstoffen

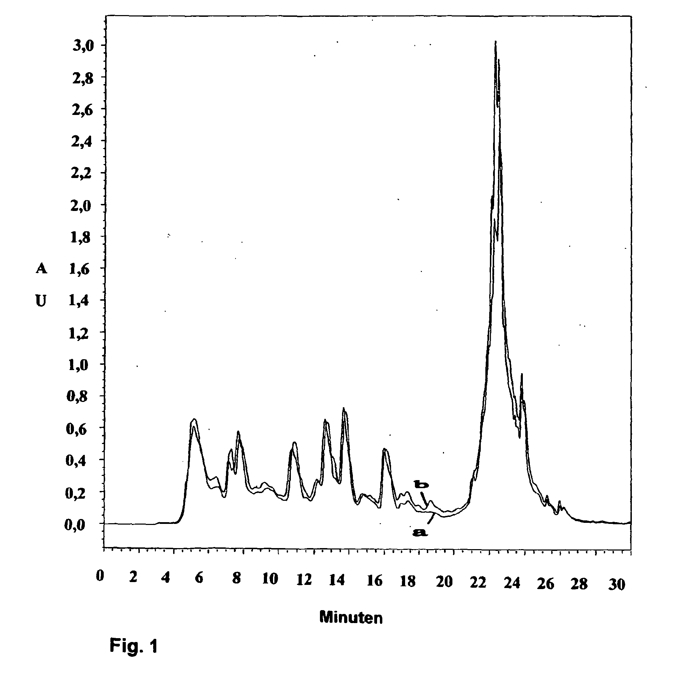

Jeweils 20 g von 5 verschiedenen pflanzlichen Ausgangsstoffen chinesischer Tees (Bambusae Caulis in Taeniam, Bupleuri Radix, Citri reticulatae Pericarpium, Codonopsis Radix, Menthae Herba) werden zu einer Mischung vereint und anschließend, wie unter Beispiel 1 beschrieben, gekocht und vom entstandenen Dekokt eine Probe entnommen, um diese mittels HPLC zu analysieren. In Figur 1 wird das erhaltene Chromatogramm (Graph a), Analyse bei 254 nm) im Vergleich mit der aus Beispiel 3 erhaltenen Analyse gezeigt.

### Beispiel 3: Getrennte Zubereitung von pflanzlichen Ausgangsstoffen

Die Beispiel 2 genannten pflanzlichen Ausgangsstoffe werden separat gemäß dem in Beispiel 1 erläuterten Verfahren gekocht und die einzelnen Dekokte anschließend vereint und mittels HPLC überprüft. Dabei entsprechen die Mengenverhältnisse jenen der Lösung des vorgehenden Beispiels. In Figur 1 wird das erhaltene Chromatogramm (Graph b), Analyse bei 254 nm) im Vergleich mit der aus Beispiel 2 erhaltenen Analyse gezeigt.

### Beispiel 4: Dekokt eines pflanzlichen Ausgangsstoffes

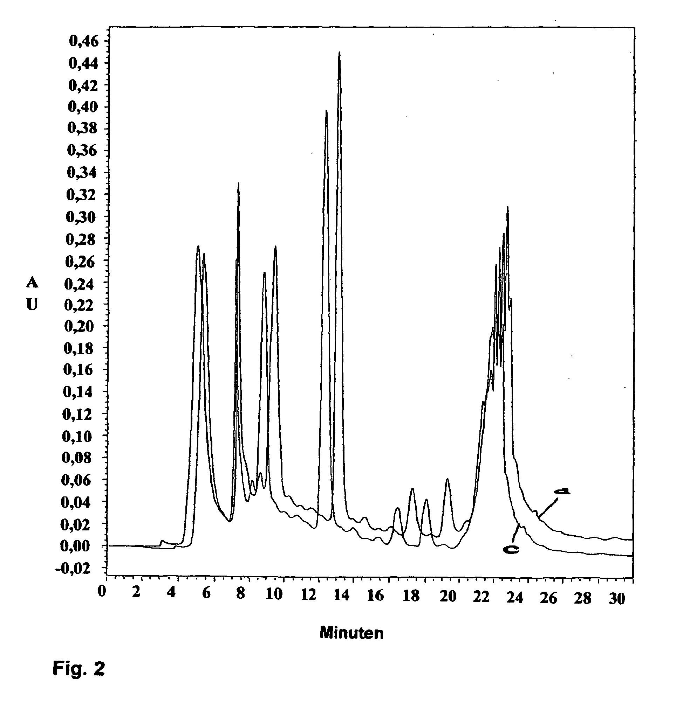

Von einem einzelnen pflanzlichen Ausgangsstoff (Artemisiae Scopariae Herba) wurde ein Dekokt gemäß dem Verfahren von Beispiel 1 hergestellt und das Dekokt anschließend mittels einer HPLC analysiert. In Figur 2 wird das erhaltene Chromatogramm (Graph c), Analyse bei 254 nm) im Vergleich mit der aus Beispiel 5 erhaltenen Analyse gezeigt.

### Beispiel 5: Dekokt und Pelletierung eines pflanzlichen Ausgangsstoffes

Ein Teil des aus Beispiel 4 hergestellten Dekoktes wird entsprechend des erfindungsgemäßen Verfahrens zu Pellets gefriergetrocknet und die Pellets anschließend in einer entsprechenden Menge Wasser gelöst und diese Lösung mittels einer HPLC analysiert. Die Mengenverhältnisse dieser Lösung entsprechen jenen der vorhergehenden Lösung. In Figur 2 wird das erhaltene Chromatogramm (Graph d), Analyse bei 254 nm) im Vergleich mit der aus Beispiel 4 erhaltenen Analyse gezeigt.

### Auswertung

Figur 1 zeigt zwei übereinandergelegte Chromatogramme der aus Beispiel 2 bzw. Beispiel 3 erhaltenen Dekokte, die mittels einer HPLC analysiert wurden. Die Identität der beiden Graphen zeigt, dass die unterschiedliche Behandlung der pflanzlichen Ausgangsstoffe zu keinerlei Unterschieden in den Mengenverhältnissen und Zusammensetzungen der Inhaltsstoffe führt. Damit ist gezeigt, dass mit den erfindungsgemäßen Pellets und dem erfindungsgemäßen Verfahren zur Herstellung der Pellets chinesische Tees in gleicher Qualität wie die der herkömmlichen Zubereitungen hergestellt werden können und gleichzeitig die zuvor erwähnten Nachteile der herkömmlichen Zubereitung von chinesischen Tees überwunden werden können.

Figur 2 zeigt die übereinandergelegten Chromatogramme der aus Beispiel 4 und Beispiel 5 erhaltenen Lösungen. Auch hierbei wird gezeigt, dass eine aus den erfindungsgemäßen Pellets zubereitete Lösung, die gleiche Zusammensetzung und Mengenverhältnisse der Inhaltsstoffe aufweist, wie ein herkömmlich zubereitetes Dekokt.

## Patentansprüche

1. Eine Zusammenstellung oder ein Kit von Pellets zur Zubereitung von chinesischen Arzneimitteltees, wobei Pellets der gleichen Sorte in einer Matrix einen Auszug jeweils eines Ausgangsstoffes enthalten, wobei der Ausgangsstoff ausgewählt ist aus einer Gruppe von verschiedenen Ausgangstoffen chinesischer Arzneimitteltees, wobei die Zusammenstellung mindestens zwei, vorzugsweise mindestens vier verschiedene jeweils voneinander getrennt vorliegende Pelletsorten enthält.

2. Eine Mischung von Pellets, wobei die Mischung mindestens zwei verschiedene Pelletsorten enthält und wobei eine Pelletsorte so definiert ist, dass der in den Pellets einer Sorte vorliegende Auszug aus jeweils einem Ausgangstoff chinesischer Arzneimitteltees gewonnen wurde.

3. Die Zusammenstellung, das Kit oder die Mischung nach Anspruch 1 oder 2, **dadurch gekennzeichnet, dass** die Pellets einer Sorte in einer Matrix einen Auszug jeweils eines Ausgangsstoffes chinesischer Arzneimitteltees enthält, wobei die Matrix einen Gerüstbildner aus hydrophilen Makromolekülen umfasst, und wobei der Ausgangsstoff chinesischer Arzneimitteltees ausgewählt ist aus der Gruppe pflanzlicher Ausgangsstoff, tierischer Ausgangsstoff, mineralischer Ausgangsstoff.

4. Die Zusammenstellung, das Kit oder die Mischung nach einem der Ansprüche 1 bis 3, **dadurch gekennzeichnet, dass** der Ausgangsstoff, aus dem der in den Pellets gleicher Sorte vorliegende Auszug gewonnen wird, höchstens ein Ausgangsstoff von mindestens zwei, vorzugsweise von mindestens vier, besonders bevorzugt von mindestens sechs, ganz besonders bevorzugt von mindestens zehn Ausgangsstoffen chinesischer Arzneimitteltees ist.

5. Die Zusammenstellung, das Kit oder die Mischung nach einem der Ansprüche 1 bis 4, **dadurch gekennzeichnet, dass** das Pellet die Instantform eines Ausgangsstoffes chinesischer Arzneimitteltees darstellt.

6. Die Zusammenstellung, das Kit oder die Mischung nach einem der Ansprüche 1 bis 5, **dadurch gekennzeichnet, dass** der Auszug eines Ausgangstoffes vor Einbringung in das Pellet durch Abkochen gewonnen wurde.

7. Die Zusammenstellung, das Kit oder die Mischung nach einem der Ansprüche 1 bis 6, **dadurch gekennzeichnet, dass** die Ausgangstoffe Drogen zur Anwendung in der Traditionellen Chinesischen Medizin darstellen.

8. Die Zusammenstellung, das Kit oder die Mischung nach einem der Ansprüche 1 bis 7, **dadurch gekennzeichnet, dass** die pflanzlichen Ausgangstoffe ausgewählt sind aus der Gruppe Pflanzen, Algen, Pilzen, Flechten oder deren Teile.

9. Die Zusammenstellung, das Kit oder die Mischung nach einem der Ansprüche 1 bis 8, **dadurch gekennzeichnet, dass** die pflanzlichen Ausgangstoffe ausgewählt sind aus den taxonomischen Gruppen Alismataceae, Companulaceae, Compositae, Cyperaceae, Dioscoreaceae, Labiatae, Leguminosae, Magnoliaceae, Polygonaceae, Ranunculaceae, Rutaceae, Rhamnaceae, Scrophulariaceae, Solanaceae, Umbelliferae.

10. Die Zusammenstellung, das Kit oder die Mischung nach einem der Ansprüche 1 bis 9, **dadurch gekennzeichnet, dass** die pflanzlichen Ausgangstoffe ausgewählt sind aus den in Tabelle 1 aufgeführten Ausgangsstoffen.

11. Die Zusammenstellung, das Kit oder die Mischung nach einem der Ansprüche 1 bis 10, **dadurch gekennzeichnet, dass** der in Pellets gleicher Sorte enthaltene Auszug eines Ausgangstoffes die Substanz oder die Kombination von Substanzen jeweils einer der in Tabelle 2 aufgeführten Gruppen enthält.

12. Die Zusammenstellung, das Kit oder die Mischung nach einem der Ansprüche 1 bis 11, **dadurch gekennzeichnet, dass** der Gerüstbildner ausgewählt ist aus der Gruppe Kollagen, Gelatine, fraktionierte Gelatine, Kollagenhydrolysate, Gelantinederivate, Pflanzenproteine, Pflanzenproteinhydrolysate, Elastinhydrolysate, Albumin, Agar-Agar, Gummi arabicum, Pektine, Traganth, Xanthan, natürliche und modifizierte Stärken, Dextrane, Dextrine, Maltodextrin, Chitosan, Alginate, Cellulosederivate, Zucker, Glycin, Lactose, Mannit, Polyvinylpyrrolidon, Polyacrylsäure, Polymere aus Methacrylsäure, Polymere aus Methacrylsäureestern, Celluloseactatphthalat, Hydroxypropylmethylcellulosephthalat, azovernetzte Polymethacrylate, Polyurethan-Zucker-Copolymere, Galactomannanderivate, Polysaccharide, Mono-, Di-, und Triglyceride und deren Mischungen.

13. Die Zusammenstellung, das Kit oder die Mischung nach einem der Ansprüche 1 bis 12, **dadurch gekennzeichnet, dass** der Gerüstbildner ausgewählt ist aus der Gruppe Oligosaccharide, Polysaccharide, Dextran, Dextrin, Maltodextrin, bevorzugt Maltodextrin oder Dextran, oder eine Mischung davon, besonders bevorzugt Dextran.

14. Die Verwendung der Zusammenstellung, des Kits oder der Mischung nach den Ansprüchen 1 bis 13 oder der Instantzubereitung zur Zubereitung von chinesischen Arzneimitteltees.

15. Ein Verfahren zur Zubereitung von chinesischen Arzneimitteltees, wobei die Ausgangsstoffe dieser chinesischen Arzneimitteltees jeweils getrennt abgekocht werden, das jeweilig erhaltende Dekokt mit Gerüstbildner versetzt wird, pelletiert wird und anschließend gefriergetrocknet wird, um nach Ausgangsstoffen getrennte Pelletsorten zu erhalten, und anschließend eine Mischung von Pellets von mindestens zwei verschiedenen Sorten in Wasser gelöst wird, um chinesischen Arzneimitteltee herzustellen.

16. Ein Verfahren zur Herstellung von chinesischen Arzneimitteltees in Instantform auf Basis von Pellets enthaltend Auszüge von Ausgangsstoffen chinesischer Arzneimitteltees, wobei der Ausgangsstoff chinesischer Arzneimitteltees ausgewählt ist aus der Gruppe pflanzlicher Ausgangsstoff, tierischer Ausgangsstoff, mineralischer Ausgangsstoff, wobei Pellets von mindestens zwei verschiedenen Sorten getrennt nach Ausgangsstoffen verwendet werden, umfassend die Schritte:
a) Zubereitung von Dekokten getrennt nach den Ausgangstoffen chinesischer Arzneimitteltees,
b) Lösen und/oder Dispergieren eines Gerüstbildners im jeweiligen Dekokt und Erhalten einer Dekokt/Gerüstbildner-Mischung,
c) Gefrieren der in Schritt b) erhaltenen Dekokt/Gerüstbildner-Mischung durch tropfenweises Einbringen in eine tiefkalte kryogene Flüssigkeit und Erhalten von Pellets,
d) Gefriertrocknung der erhaltenen Pellets.

17. Das Verfahren nach Anspruch 16, **dadurch gekennzeichnet, dass** nach Schritt a) Feststoffe, vorzugsweise durch Filtration entfernt werden.

18. Das Verfahren nach Ansprüche 16 oder 17, **dadurch gekennzeichnet, dass** es sich bei den zu verarbeitenden Ausgangsstoffen um pflanzliche, tierische oder mineralische Drogen, zur Anwendung in der traditionellen Chinesischen Medizin handelt.

19. Das Verfahren nach einem der Ansprüche 16 bis 18, **dadurch gekennzeichnet, dass** als pflanzliche Ausgangstoffe Pflanzen, Algen, Pilzen, Flechten oder deren Teile verwendet werden.

20. Das Verfahren nach einem der Ansprüche 16 bis 19, **dadurch gekennzeichnet, dass** die pflanzlichen Ausgangstoffe ausgewählt werden aus den taxonomischen Gruppen Alismataceae, Companulaceae, Compositae, Cyperaceae, Dioscoreaceae, Labiatae, Leguminosae, Magnoliaceae, Polygonaceae, Ranunculaceae, Rutaceae, Rhamnaceae, Scrophulariaceae, Solanaceae, Umbelliferae.

21. Das Verfahren nach einem der Ansprüche 16 bis 20, **dadurch gekennzeichnet, dass** die Ausgangstoffe definiert sind durch die Substanz oder die Kombination von Substanzen jeweils einer der in Tabelle 2 aufgeführten Gruppen.

22. Das Verfahren nach einem der Ansprüche 16 bis 21, **dadurch gekennzeichnet, dass** das Gewichtsverhältnis von eingesetzter Festsubstanz der pflanzlichen Ausgangsstoffe und Wasser zu dem in Schritt a) erhaltenen Dekokt 0,8:1 bis 1,2:1, bevorzugt, 0,9:1 bis 1,1:1, besonders bevorzugt um 1:1 beträgt.

23. Das Verfahren nach einem der Ansprüche 16 bis 22, **dadurch gekennzeichnet, dass** der Feststoffgehalt des in Schritt a) erhaltenen und in Schritt b) weiter zu verarbeitenden Dekoktes 1 bis 20 Gew.-%, vorzugsweise 1 bis 10 Gew.-% beträgt.

24. Das Verfahren nach einem der Ansprüche 16 bis 23, **dadurch gekennzeichnet, dass** der Gerüstbildner ausgewählt ist aus der Gruppe Kollagen, Gelatine, fraktionierte Gelatine, Kollagenhydrolysate, Gelantinederivate, Pflanzenproteine, Pflanzenproteinhydrolysate, Elastinhydrolysate, Albumin, Agar-Agar, Gummi arabicum, Pektine, Traganth, Xanthan, natürliche und modifizierte Stärken, Dextrane, Dextrine, Maltodextrin, Chitosan, Alginate, Cellulosederivate, Zucker, Glycin, Lactose, Mannit, Polyvinylpyrrolidon, Polyacrylsäure, Polymere aus Methacrylsäure, Polymere aus Methacrylsäureestern, Celluloseactatphthalat, Hydroxypropylmethylcellulosephthalat, azovernetzte Polymethacrylate, Polyurethan-Zucker-Copolymere, Galactomannanderivate, Polysaccharide, Mono-, Di-, und Triglyceride und deren Mischungen.

25. Das Verfahren nach einem der Ansprüche 16 bis 24, **dadurch gekennzeichnet, dass** der Gerüstbildner ausgewählt ist aus der Gruppe Oligosaccharide, Polysaccharide, Dextran, Dextrin, Maltodextrin.

26. Das Verfahren nach einem der Ansprüche 16 bis 25, **dadurch gekennzeichnet, dass** nach Lösen des Gerüstbildners in Schritt b) 2 - 50 Gew.-%, vorzugsweise 5 - 40 Gew.-% Gerüstbildner in der Dekokt/Gerüstbildner-Mischung vorliegt.

27. Instantform eines Ausgangsstoffes chinesischer Arzneimitteltees in Form von gefriergetrockneten nach Ausgangsstoffen getrennten Pelletsorten erhältlich nach einem Verfahren gemäß einem der Ansprüche 16 bis 26.

## Claims

1. A composite or a kit of pellets for the preparation of Chinese medicinal teas, wherein pellets of the same type include in a matrix an extract of one starting material at a time, said starting material being selected from a group of different starting materials of Chinese medicinal teas, said composite including at least two, preferably at least four different types of pellets, each one separated from each other.

2. A mixture of pellets, said mixture including at least two different types of pellets, with one type of pellets being defined such that the extract present in the pellets of one type has been obtained from one starting material of Chinese medicinal teas at a time.

3. The composite, the kit, or the mixture according to claim 1 or 2, **characterized in that** the pellets of one type include in a matrix an extract of one starting material of Chinese medicinal teas at a time, said matrix comprising a structure-forming agent made up of hydrophilic macromolecules, and the starting material of Chinese medicinal teas being selected from the group of vegetable starting material, animal starting material, mineral starting material.

4. The composite, the kit, or the mixture according to any of claims 1 to 3,
**characterized in that** the starting material from which the extract present in the pellets of the same type is obtained is at most one starting material of at least two, preferably at least four, more preferably at least six, and especially preferably at least ten starting materials of Chinese medicinal teas.

5. The composite, the kit, or the mixture according to any of claims 1 to 4, **characterized in that** the pellet represents the instant form of a starting material of Chinese medicinal teas.

6. The composite, the kit, or the mixture according to any of claims 1 to 5, **characterized in that** the extract of a starting material is obtained by boiling prior to incorporation in the pellet.

7. The composite, the kit, or the mixture according to any of claims 1 to 6, **characterized in that** the starting materials represent drugs for use in traditional Chinese medicine.

8. The composite, the kit, or the mixture according to any of claims 1 to 7, **characterized in that** the vegetable starting materials are selected from the group of plants, algae, fungi, lichens or parts thereof.

9. The composite, the kit, or the mixture according to any of claims 1 to 8, **characterized in that** the vegetable starting materials are selected from the taxonomic groups of Alismataceae, Companulaceae, Compositae, Cyperaceae, Dioscoreaceae, Labiatae, Leguminosae, Magnoliaceae, Polygonaceae, Ranunculaceae, Rutaceae, Rhamnaceae, Scrophulariaceae, Solanaceae, Umbelliferae.

10. The composite, the kit, or the mixture according to any of claims 1 to 9, **characterized in that** the vegetable starting materials are selected from the starting materials listed in Table 1.

11. The composite, the kit, or the mixture according to any of claims 1 to 10, **characterized in that** the extract of a starting material included in pellets of the same type includes the substance or combination of substances of one of the groups at a time that are listed in Table 2.

12. The composite, the kit, or the mixture according to any of claims 1 to 11, **characterized in that** the structure-forming agent is selected from the group of collagen, gelatin, fractionated gelatin, collagen hydrolysates, gelatin derivatives, vegetable proteins, vegetable protein hydrolysates, elastin hydrolysates, albumin, agar, gum arabic, pectins, tragacanth, xanthan, natural and modified starches, dextrans, dextrins, maltodextrin, chitosan, alginates, cellulose derivatives, sugars, glycine, lactose, mannitol, polyvinylpyrrolidone, polyacrylic acid, polymers of methacrylic acid, polymers of methacrylic esters, cellulose acetate phthalate, hydroxypropylmethylcellulose phthalate, azo-crosslinked polymethacrylates, polyurethane-sugar copolymers, galactomannan derivatives, polysaccharides, mono-, di-, and triglycerides, and mixtures thereof.

13. The composite, the kit, or the mixture according to any of claims 1 to 12, **characterized in that** the structure-forming agent is selected from the group of oligosaccharides, polysaccharides, dextran, dextrin, maltodextrin, preferably maltodextrin or dextran, or a mixture thereof, especially preferably dextran.

14. Use of the composite, the kit, or the mixture according to any of claims 1 to 13 or of the instant preparation for the preparation of Chinese medicinal teas.

15. A method of preparing Chinese medicinal teas, wherein each of the starting materials of said Chinese medicinal teas is boiled separately, the decoction obtained in each case is added with structure-forming agent, pelletized, and subsequently freeze-dried to obtain types of pellets separated according to starting materials, and a mixture of pellets of at least two different types is subsequently dissolved in water to produce Chinese medicinal tea.

16. A method for the production of Chinese medicinal teas in instant form on the basis of pellets containing extracts of starting materials of Chinese medicinal teas, wherein the starting material of Chinese medicinal teas is selected from the group of vegetable starting material, animal starting material, mineral starting material, and wherein pellets of at least two different types are used, separated according to starting materials, said method comprising the steps of:
a) preparing decoctions separately according to the starting materials of Chinese medicinal teas,
b) dissolving and/or dispersing a structure-forming agent in each decoction to obtain a mixture of decoction/structure-forming agent,
c) freezing the mixture of decoction/structure-forming agent obtained in step b) by dripping into a very cold cryogenic liquid to obtain pellets,
d) freeze-drying the pellets obtained.

17. The method according to claim 16, **characterized in that** solids are removed after step a), preferably by filtration.

18. The method according to claim 16 or 17, **characterized in that** the starting materials to be processed are vegetable, animal or mineral drugs for use in traditional Chinese medicine.

19. The method according to any of claims 16 to 18, **characterized in that** plants, algae, fungi, lichens or parts thereof are used as vegetable starting materials.

20. The method according to any of claims 16 to 19, **characterized in that** the vegetable starting materials are selected from the taxonomic groups of Alismataceae, Companulaceae, Compositae, Cyperaceae, Dioscoreaceae, Labiatae, Leguminosae, Magnoliaceae, Polygonaceae; Ranunculaceae, Rutaceae, Rhamnaceae, Scrophulariaceae, Solanaceae, Umbelliferae.

21. The method according to any of claims 16 to 20, **characterized in that** each starting material is defined by the substance or combination of substances of one of the groups listed in Table 2.

22. The method according to any of claims 16 to 21, **characterized in that** the weight ratio of employed solids of the vegetable starting materials and water to decoction obtained in step a) is from 0.8:1 to 1.2:1, preferably from 0.9:1 to 1.1:1, more preferably around 1:1.

23. The method according to any of claims 16 to 22, **characterized in that** the solids content of the decoction obtained in step a) and to be further processed in step b) is from 1 to 20 wt.-%, preferably from 1 to 10 wt.-%.

24. The method according to any of claims 16 to 23, **characterized in that** the structure-forming agent is selected from the group of collagen, gelatin, fractionated gelatin, collagen hydrolysates, gelatin derivatives, vegetable proteins, vegetable protein hydrolysates, elastin hydrolysates, albumin, agar, gum arabic, pectins, tragacanth, xanthan, natural and modified starches, dextrans, dextrins, maltodextrin, chitosan, alginates, cellulose derivatives, sugars, glycine, lactose, mannitol, polyvinylpyrrolidone, polyacrylic acid, polymers of methacrylic acid, polymers of methacrylic esters, cellulose acetate phthalate, hydroxypropylmethylcellulose phthalate, azo-crosslinked polymethacrylates, polyurethane-sugar copolymers, galactomannan derivatives, polysaccharides, mono-, di-, and triglycerides, and mixtures thereof.

25. The method according to any of claims 16 to 24, **characterized in that** the structure-forming agent is selected from the group of oligosaccharides, polysaccharides, dextran, dextrin, maltodextrin.

26. The method according to any of claims 16 to 25, **characterized in that** after dissolving the structure-forming agent in step b), 2 to 50 wt.-%, preferably 5 to 40 wt.-% of structure-forming agent is present in the mixture of decoction/structure-forming agent.

27. An instant form of a starting material of Chinese medicinal teas in the form of freeze-dried types of pellets separated according to the starting materials, which can be obtained by means of a method according to any of claims 16 to 26.

## Revendications

1. Assortiment ou ensemble de granulés pour la préparation de tisanes médicinales chinoises, dans lequel des granulés du même type contiennent chacun un extrait d'une substance de départ dans une matrice, laquelle substance de départ est choisie dans un groupe de différentes substances de départ pour tisanes médicinales chinoises, l'assortiment contenant au moins deux, de préférence au moins quatre types de granulés différents présents séparés les uns des autres.

2. Mélange de granulés qui contient au moins deux types de granulés différents et dans lequel un type de granulé est ainsi défini que chaque extrait présent dans les granulés d'un type a été tiré d'une substance de départ pour tisanes médicinales chinoises.

3. Assortiment, ensemble ou mélange selon la revendication 1 ou la revendication 2, **caractérisé en ce que** les granulés d'un type contiennent dans une matrice un extrait respectif d'une substance de départ pour tisanes médicinales chinoises, laquelle matrice comprend un générateur de structure à base de macromolécules hydrophiles, et laquelle substance de départ pour tisanes médicinales chinoises est choisie dans le groupe des substances de départ végétales, des substances de départ animales et des substances de départ minérales.

4. Assortiment, ensemble ou mélange selon l'une des revendications 1 à 3, **caractérisé en ce que** la substance de départ à partir de laquelle l'extrait présent dans les granulés de même type est obtenu est au plus une substance de départ d'au moins deux, de préférence au moins quatre, de façon particulièrement préférée au moins six, de façon tout particulièrement préférée au moins dix substances de départ pour tisanes médicinales chinoises.

5. Assortiment, ensemble ou mélange selon l'une des revendications 1 à 4,
**caractérisé en ce que** le granulé représente la forme instantanée d'une substance de départ pour tisanes médicinales chinoises.

6. Assortiment, ensemble ou mélange selon l'une des revendications 1 à 5, **caractérisé en ce que** l'extrait d'une substance de départ a été obtenu par décoction avant d'être incorporé dans le granulé.

7. Assortiment, ensemble ou mélange selon l'une des revendications 1 à 6, **caractérisé en ce que** les substances de départ représentent des drogues destinées à être utilisées dans la médecine traditionnelle chinoise.

8. Assortiment, ensemble ou mélange selon l'une des revendications 1 à 7, **caractérisé en ce que** les substances de départ végétales sont choisies dans le groupe comprenant des plantes, des algues, des champignons, des lichens ou des parties de ces végétaux.

9. Assortiment, ensemble ou mélange selon l'une des revendications 1 à 8, **caractérisé en ce que** les substances de départ végétales sont choisies dans les groupes taxonomiques des alismatacées, campanulacées, composées, cypéracées, dioscoréacées, labiées, légumineuses, magnoliacées, polygonacées, renonculacées, rutacées, rhamnacées, scrofulariacées, solanacées, ombellifères.

10. Assortiment, ensemble ou mélange selon l'une des revendications 1 à 9, **caractérisé en ce que** les substances de départ végétales sont choisies parmi les substances de départ énumérées dans le tableau 1.

11. Assortiment, ensemble ou mélange selon l'une des revendications 1 à 10, **caractérisé en ce que** l'extrait d'une substance de départ contenu dans des granulés de même type contient la substance ou la combinaison de substances de l'un des groupes indiqués dans le tableau 2.

12. Assortiment, ensemble ou mélange selon l'une des revendications 1 à 11,
**caractérisé en ce que** le générateur de structure est choisi dans le groupe comprenant le collagène, des gélatines, des gélatines fractionnées, des hydrolysats de collagène, des dérivés de gélatine, des protéines végétales, des hydrolysats de protéines végétales, des hydrolysats d'élastine, l'albumine, l'agar-agar, la gomme arabique, des pectines, la gomme adragante, la gomme de xanthane, des amidons naturels et modifiés, des dextranes, des dextrines, la maltodextrine, le chitosane, des alginates, des dérivés de cellulose, des sucres, la glycine, le lactose, le mannitol, la polyvinylpyrrolidone, l'acide polyacrylique, des polymères de l'acide méthacrylique, des polymères d'esters de l'acide méthacrylique, l'acétate phtalate de cellulose, le phtalate d'hydroxypropylméthylcellulose, des polyméthacrylates azo-réticulés, des copolymères de polyuréthane et de sucre, des dérivés de galactomannane, des polysaccharides, des mono-, di- et triglycérides, ainsi que leurs mélanges.

13. Assortiment, ensemble ou mélange selon l'une des revendications 1 à 12, **caractérisé en ce que** le générateur de structure est choisi dans le groupe comprenant des oligosaccharides, des polysaccharides, le dextrane, la dextrine, la maltodextrine, de préférence la maltodextrine ou le dextrane, ou un mélange de ceux-ci, de façon particulièrement préférée le dextrane.

14. Utilisation de l'assortiment, ensemble ou mélange selon les revendications 1 à 13 ou de la préparation instantanée pour la préparation de tisanes médicinales chinoises.

15. Procédé de préparation de tisanes médicinales chinoises, dans lequel une décoction de chacune des substances de départ de ces tisanes médicinales chinoises est préparée séparément, chaque décoction est mélangée avec le générateur de structure, granulée et ensuite lyophilisée afin d'obtenir des types de granulés séparés par substances de départ, puis un mélange de granulés d'au moins deux types différents est dissous dans de l'eau afin de préparer une tisane médicinale chinoise.

16. Procédé de préparation de tisanes médicinales chinoises sous forme instantanée à base de granulés contenant des extraits de substances de départ pour tisanes médicinales chinoises, dans lequel la substance de départ pour tisanes médicinales chinoises est choisie dans le groupe des substances de départ végétales, des substances de départ animales et des substances de départ minérales, des granulés d'au moins deux types différents séparés par substances de départ étant utilisés, ledit procédé comprenant les étapes consistant à :
a) Préparer des décoctions séparées par substances de départ pour tisanes médicinales chinoises ;
b) Dissoudre et/ou disperser un générateur de structures dans chaque décoction afin d'obtenir un mélange décoction/générateur de structure ;
c) Congeler le mélange décoction/générateur de structure obtenu à l'étape b) en le versant goutte à goutte dans un liquide cryogénique à très basse température afin d'obtenir des granulés ;
d) Lyophiliser les granulés obtenus.

17. Procédé selon la revendication 16, **caractérisé en ce que**, après l'étape a), les solides sont enlevés, de préférence par filtration.

18. Procédé selon la revendication 16 ou la revendication 17, **caractérisé en ce que** les substances de départ mises en oeuvre sont des drogues végétales, animales ou minérales destinées à être employées dans la médecine traditionnelle chinoise.

19. Procédé selon l'une des revendications 16 à 18, **caractérisé en ce que** les substances de départ utilisées sont des plantes, des algues, des champignons, des lichens ou des parties de ces végétaux.

20. Procédé selon l'une des revendications 16 à 19, **caractérisé en ce que** les substances de départ végétales sont choisies dans les groupes taxonomiques des alismatacées, campanulacées, composées, cypéracées, dioscoréacées, labiées, légumineuses, magnoliacées, polygonacées, renonculacées, rutacées, rhamnacées, scrofulariacées, solanacées, ombellifères.

21. Procédé selon l'une des revendications 16 à 20, **caractérisé en ce que** les substances de départ sont définies par la substance ou la combinaison de substances de l'un des groupes indiqués dans le tableau 2.

22. Procédé selon l'une des revendications 16 à 21, **caractérisé en ce que** le ratio en poids de la matière solide des substances de départ de départ utilisées et de l'eau sur la décoction obtenue à l'étape a) est compris entre 0,8:1 à 1,2:1, de préférence entre 0,9:1 et 1,1:1 et de façon particulièrement préférée voisin de 1:1.

23. Procédé selon l'une des revendications 16 à 22, **caractérisé en ce que** la teneur en solides de la décoction obtenue à l'étape a) et retraitée à l'étape b) se situe dans la plage de 1 à 20% en poids, de préférence de 1 à 10% en poids.

24. Procédé selon l'une des revendications 16 à 23, **caractérisé en ce que** le générateur de structure est choisi dans le groupe comprenant le collagène, des gélatines, des gélatines fractionnées, des hydrolysats de collagène, des dérivés de gélatine, des protéines végétales, des hydrolysats de protéines végétales, des hydrolysats d'élastine, l'albumine, l'agar-agar, la gomme arabique, des pectines, la gomme adragante, la gomme de xanthane, des amidons naturels et modifiés, des dextranes, des dextrines, la maltodextrine, le chitosane, des alginates, des dérivés de cellulose, des sucres, la glycine, le lactose, le mannitol, la polyvinylpyrrolidone, l'acide polyacrylique, des polymères de l'acide méthacrylique, des polymères d'esters de l'acide méthacrylique, l'acétate phtalate de cellulose, le phtalate d'hydroxypropylméthylcellulose, des polyméthacrylates azo-réticulés, des copolymères de polyuréthane et de sucre, des dérivés de galactomannane, des polysaccharides, des mono-, di- et triglycérides, ainsi que leurs mélanges.

25. Procédé selon l'une des revendications 16 à 24, **caractérisé en ce que** le générateur de structure est choisi dans le groupe comprenant des oligosaccharides, des polysaccharides, le dextrane, la dextrine, la maltodextrine.

26. Procédé selon l'une des revendications 16 à 25, **caractérisé en ce que**, après dissolution du générateur de structure à l'étape b), le mélange décoction/générateur de structure contient de 2 à 50% en poids, de préférence 5 à 40% en poids de générateur de structure.

27. Forme instantanée d'une substance de départ pour tisanes médicinales chinoises sous la forme de types de granulés lyophilisés séparés par substances de départ qui peuvent être obtenus par un procédé selon l'une des revendications 16 à 26.
